# EUROPEAN PATENT SPECIFICATION

(11) **EP 1 029 881 B1**
(45) Date of publication and mention of the grant of the patent: **12.05.2004**
(21) Application number: 00201220.1
(22) Date of filing: 20.10.1993
(51) Int. Cl.: C08G 65/10, C08G 65/26, C08G 65/32, C07D 307/08

(54) **Depolymerization to cyclic ethers using selected metal compound catalysts**
Depolymerisation zu zyclischen Ethern unter Verwendung der Katalysatoren aus gewählten Metallverbindungen
Dépolymérisation pour obtenir des éthers cycliques à l'aide de la catalyse par des composés métalliques choisis

(30) Priority: 21.10.1992 US 964313; 23.02.1993 US 21368; 16.07.1993 US 93243
(43) Date of publication of application: 23.08.2000
(62) Divisional of application: 93924316.8
(73) Proprietor: E.I. DU PONT DE NEMOURS AND COMPANY, Wilmington, Delaware 19898 (US)
(72) Inventor: Drysdale, Neville Everton, Newark, DE 19702-1026 (US); Bockrath, Richard Edmund, Wilmington, DE 19806-1331 (US)
(74) Representative: Carpmaels & Ransford

(56) References cited:
- WO-A-88/02661
- US-A- 4 115 408
- US-A- 4 153 786
- P. DREYFUSS ET AL.:: "Graft copolymers by oxonium polymerization" POLYMER LETTERS ED., vol. 14, 1976, pages 139-142, XP002163269
- CHEMICAL ABSTRACTS, vol. 78, no. 16, 23 April 1973 (1973-04-23) Columbus, Ohio, US; abstract no. 98454j, MATSUKURA K. ET AL.:: "Polyether esters produced by using yttrium catalysts" XP002163270 & JP 07 231438 A (UNITIKA CO. LTD) 29 August 1995 (1995-08-29)
- DATABASE WPI Week 198745 Derwent Publications Ltd., London, GB; AN 1987-337120 XP002163271 & JP 62 240319 A (MITSUBISHI CHEM. IND. K.K.), 21 October 1987 (1987-10-21)
- DATABASE WPI Week 198625 Derwent Publications Ltd., London, GB; AN 1986-187083 XP002163272 & JP 61 120832 A (ASAHI CHEMICAL IND. K.K.), 7 June 1986 (1986-06-07)

## Description

### FIELD OF THE INVENTION

This invention concerns the depolymerization of polytetrahydrofurans to tetrahydrofurans, catalyzed by selected metal compounds.

### BACKGROUND OF THE INVENTION

Cyclic ethers are polymerized by various means to give products of widespread utility. For instance, ethylene oxide is polymerized to polyethylene oxide which is useful, in lower molecular weight grades, for ceramics (as a binder), cosmetics, lubricants, polyurethanes; and in higher molecular weight grades, for packaging film, denture adhesives, lubricants, flocculation and for other articles and products. Tetrahydrofuran (THF) is polymerized to poly(tetramethylene ether) glycol which is useful in the preparation of Spandex fibres; polyurethane resins which are useful in elastomeric parts; and thermoplastic elastomers which are useful for molding various mechanical parts. Therefore, improved methods of making these polymers are sought. Also useful are methods of depolymerizing the polyethers to useful products, such as the cyclic ethers from which they were originally made. Such depolymerizations allow for the recycle of off specification or used polyethers to useful products such as polyethers; thereby reducing waste.

U.S. Patent 3,842,019 describes the polymerization of oxiranes and other small ring compounds by a presumed cationic mechanism, using as the catalyst the decomposition products of metal perfluoroalkylsulfonates. These catalysts are described as "latent", that is no reaction occurs until the metal salt is decomposed. The reactions reported are relatively slow, even at elevated temperatures.

U.S. Patents 5,084,586 and 5,124,417 describe the cationic polymerization of various monomers, including cyclic ethers, using onium cations, whose corresponding anions are fluororalkylsulfatometallates. Onium ion catalyzed cationic polymerizations are well known, and there is no mention in these patents of the use of metal salts not containing onium ions, such as metal triflates, as catalysts for the polymerization of cyclic ethers.

Japanese Patent Application 51-82397 describes the polymerization of tetrahydrofuran using a combination of fluorosulfonic acid and a carboxylic acid as catalysts. No mention is made of metal salts, such a metal triflates as catalysts.

J. S. Hrkach, et al., Macromolecules, vol. 23, p. 4042-4046 (1990) describe the polymerization of tetrahydrofuran using trimethylsilyl trifluoromethanesulfonate as the initiator. No mention is made of any other triflates as catalysts for this polymerization.

German Patent Application 2,459,163 describes the polymerization of THF using a combination of ferric chloride and carboxylic anhydride as catalyst.

G. A. Olah, et al., J. Appl. Polym. Sci., Vol. 45, 1355-1360 (1992) describe the use of boron, aluminum and gallium tristriflate to catalyze the polymerization of THF.

S. L. Borkowsky, et al., Organometal., Vol. 10, p. 1268-1274 (1991) report that certain zirconium complexes can initiate the polymerization of tetrahydrofuran. No mention is made of zirconium perfluoroalkylsulfonates, or of copolymers.

T. Misaki, et al., Nippon Kagaku Kaishi, p. 168-174 (1973) report on the polymerization of THF using a combination of metal aceylacetonates and acetyl chloride.

None of the metal compounds herein disclosed as catalysts are mentioned in the above references.

### SUMMARY OF THE INVENTION

This invention involves a process for the depolymerization of a polyether to a tetrahydrofuran, comprising, contacting at a temperature of about 100°C to about 250°C, a polymer consisting essentially of one or more repeat units of the formula

-[CHR¹CR²R³CR²R³CHR⁴O]-

with a compound of the formula MZₛ.Qₜ, wherein:
each R¹, R², R³ and R⁴ is independently hydrogen or hydrocarbyl containing 1 to 20 carbon atoms;

M is a metal selected from the group consisting of cobalt, vanadiam, niobium, tungsten, strontium, barium, scandium, yttrium, thulium and the other rare earth metals, titanium, zirconium, hafnium, chromium, molybdenum, tantalum, rhenium, iron, ruthenium, osmium, rhodium, iridium, palladium, platinum, gold, zinc, cadmium, mercury, copper, mischmetall, indium, silicon, germanium, tin, lead, arsenic, antimony and bismuth;

At least one of Z is an anion of the formula -OSO₂R⁵, wherein R⁵ is perfluoroalkyl containing 1 to 12 carbon atoms or part of a fluorinated polymer wherein the carbon atoms alpha and beta to the sulfonate group are together bonded to at least four fluorine atoms, or tetraphenylborate, and the remainder of Z is oxo or one or more monovalent anions;
S is 2 when M is copper, strontium, barium, cobalt, rhodium, iridium, palladium, platinum, chromium, zinc, cadmium or mercury;
S is 3 when M is scandium, yttrium, thulium or other rare earth metal, arsenic, antimony, bismuth, gold, iron, ruthenium, osmium, indium or mischmetall;
S is 4 when M is titanium, zirconium, hafnium, molybdenum, silicon, germanium, tin, or lead;
S is 5 when M is rhenium, vanadium, niobium or tantalum;
S is 6 when M is tungsten;
Q is a neutral ligand;
T is 0 or an integer of 1 to 6;
and provided that each oxo group present counts as two of s.

### DETAILS OF THE INVENTION

In the polymerization process described in the parent application EP-A-0 665 859 (published as WO-A-94/09055) one or more cyclic ethers, oxiranes, oxetanes, 1,3-dioxolanes, 1,3,5-trioxanes, or tetrahydrofurans are polymerized to form a polyether. Oxirane (more commonly called epoxide) is herein given its usual structure, a saturated three membered ring containing two carbon atoms and one oxygen atom. Oxetane is also given its common meaning, a saturated four membered ring containing three carbon atoms and one oxygen atom. The term oxepane means a saturated seven membered ring containing six carbon atoms and one oxygen atom. The term 1,3-dioxolane means a saturated five membered ring which contains two oxygen atoms separated by I carbon atom. The term 1,3,5-trioxane means a six membered ring containing 3 oxygen atoms in which the oxygen atoms and carbons atoms are alternated. All of these terms include compounds containing those ring systems which are substituted with hydrocarbyl or hydrocarbylene groups containing 1 to 20 carbon atoms. The hydrocarbylene groups may form carbocyclic rings, which include bicyclic, tricyclic, etc., systems. By a hydrocarbylene group herein is meant a divalent radical containing carbon and hydrogen which is part of a carbocyclic ring.

Preferred cyclic ethers have the formula 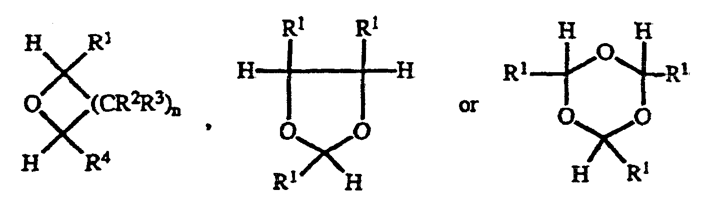 wherein n is 2 or 4 and each R¹, R², R³ and R⁴ is independently hydrogen or hydrocarbyl containing 1 to 20 carbon atoms. Some of these cyclic ethers polymerize to give repeat units of the formula -[CHR¹(CR²R³)ₙCHR⁴O]-. In a more preferred cyclic ether all of R¹, R², R³ and R⁴ are hydrogen. In another more preferred cyclic ether where n=2, R¹, one of R², both of R³ and R⁴ are hydrogen, and the remaining R² is alkyl containing 1-4 carbon atoms, especially preferably the remaining R² is methyl. By hydrocarbyl herein is meant a univalent radical containing carbon and hydrogen.

The polymerization is run in the presence of an accelerator. Suitable accelerators are carboxylic anhydrides,-acyl halides, and carboxylic acids with a pkₐ of less than about 6 in water.

By a carboxylic anhydride is meant a compound containing the grouping -C(O)O(O)C-, wherein the free valencies are to other carbon atoms. A preferred carboxylic anhydride is an anhydride of an alkyl carboxylic acid or a halogen substituted alkyl carboxylic acid, and particularly preferred anhydrides are acetic anhydride and trifluoroacetic anhydride.

By an acyl halide is meant a compound containing the grouping -C(O)X, where X is chlorine or bromine and the free valence is to another carbon atom. In preferred acyl halides, X is chlorine. Preferred acyl halides are alkyl acyl halides, and especially preferred are acetyl halides, more preferably acetyl chloride.

By a carboxylic acid is meant a compound containing the grouping -C(O)OH, wherein the free valence is to another carbon atom. Preferred carboxylic acids have a pKa of less than 5 in water. Useful carboxylic acids include, but are not limited to acetic, trifluoroacetic, chloroacetic, benzoic, trichloroacetic, p-nitrobenzoic, butyric, formic, cyanoacetic, nitropropionic, acrylic, methacrylic, napthoic acids, N-acetylglycine and N-acetyltryptophan. Preferred carboxylic acids are trifluoroacetic, acetic, formic, cyanoacetic, nitropropionic, acrylic, methacrylic acids, N-acetylglycine and N-acetyltryptophan.

When carboxylic anhydride is present one half or more of the end groups are carboxylic esters. As is known to the artisan, these may be hydrolyzed to hydroxyl groups by reaction with water, preferably in the presence of a catalyst, such as a strong acid (sulfuric acid for instance) or a strong base (such as NaOH). The proportion of acetate ends increases the longer the polymerization is allowed to proceed. Although the polymeric diol is often the desired product (it can be used to make other polymers, such as polyurethanes and polyesters), the half ester or diester is also useful, as in relatively low molecular polymers which can be used as solvents.

When an acyl halide is used as the accelerator, the end groups are usually ester on one end, and the halide, X, on the other. Thus the complete formula for such a polymer could be X-[CHR¹(CR²R³)ₙCHR⁴O]-C(O)Y, where Y is the group to which the acyl group of the acyl halide was bound. Such polymers are useful as intermediates for the preparation of polymers containing different functional groups. For example, the ester may be hydrolyzed to a hydroxyl group, and the halide may be reacted to form another functional group such as nitrile. If a bis(acyl halide), X(O)CYC(O)X, is used as the accelerator, the product of the polymerization will be a polyether with halide (X) end groups which contains two internal ester groups, and may have the formula X-[CHR¹(CR²R³)ₙCHR⁴O]-C(O)YC(O)-[OCHR¹(CR²R³)ₙCHR⁴]-X. Useful bis(acyl halides) include adipoyl chloride, terephthaloyl chloride, and diglycolyl chloride [Cl(O)CCH₂OCH₂C(O)Cl].

When a carboxylic acid is used as the accelerator, both end groups are usually mostly ester. Thus the complete formula for such a polymer could be YO-[CHR¹(CR²R³)ₙCHR⁴O]-C(O)Y, where Y is the group to which the acyl group of the carboxylic acid was bound.

An important consideration in the preparation of polyethers is the number average molecular weight (Mn) of the polyether and its molecular weight distribution. When the polyether is to be used as a monomer in the preparation of another polymer (usually in the diol form), it is often preferred that the Mn of the polyether be in the range of about 400 to about 20,000, preferably about 500 to about 5,000.

The catalyst may be yttrium or rare earth compound of the formula MZ₃ where M is a trivalent ion of yttrium, or one of the rare earths, lanthanum, cerium, praeseodymium, neodymium, promethium, samarium, europium, gadolinium, terbium, dysprosium, holmium, erbium, thulium, ytterbium, and lutetium.

Preferred metals, M, are strontium, scandium yttrium, the rare earth metals, titanium, zirconium, hafnium, vanadium, niobium, tantalum, chromium, molybdenum, tungsten, rhenium, iron, ruthenium, palladium, copper, gold, zinc, tin and bismuth. More preferred metals are yttrium, the rare earth metals, and scandium. Especially preferred metals are yttrium, ytterbium, dysprosium, erbium, neodymium, lanthanum, and scandium. Another preferred metal is "mischmetall" (sometimes also called "didymium"), which is a mixture of rare earth metals as obtained from the ore.

It is believed monovalent anions that are relatively nonnucleophilic are useful as Z. Examples of such anions are tetraphenylborate, -OSO₂R⁵, wherein R⁵ is perfluoroalkyl, or wherein R⁵ is part of a fluorinated polymer wherein the carbon atoms alpha and beta to a sulfonate group are together bonded to at least 4 fluorine atoms (as in -CF₂CF₂OSO₂-). It is preferred if R⁵ is perfluoroalkyl. In a particularly preferred R⁵ is trifluoromethyl, and the anion is herein called "triflate".

Generally speaking, any metallic compound in which the correct metal in the correct oxidation state (see above) is present and bonded to a triflate or similar anion will be a catalyst. Such a compound must of course be reasonably stable during the polymerization (or depolymerization, see below), or decompose to another compound which is still a triflate (or similar anion) compound of the metal in the correct oxidation state. It has been found that, in general, the greater the number of triflate groups bonded to the metal cation, the more active the metal compound will be as a catalyst. It is preferred if half or more of the anions (Z) bound to each metal cation is triflate or a similar anion.

The metal catalysts may optionally contain one or more neutral ligands coordinated to the metal. By a neutral ligand is meant a neutral compound that can coordinate with the catalysts, usually the metal cation. Neutral ligands include water, and ethers such as dimethyl ether and tetrahydrofuran. Useful compounds containing neutral ligands include bis(n-cyclopentadienyl)tetrahydrofuran-bis(trifluoromethanesulfonato)-zirconium and bis(n-cyclopentadienyl)tetrahydrofuran-bis(trifluoromethanesulfonato)hafnium.

The metals catalysts may contain other anions than triflate and similar anions, and tetrafluoroborate, although at least one of triflate or tetrafluoroborate anions must be present. Some other useful anions are alkoxide, particularly lower alkoxide containing 1-4 carbon atoms, acetylacetonate, cyclopentadieneide, pentamethylcyclopentadieneide, t-butylacetylacetonate, and halide. It is preferred if all of the anions are triflate.

In general, the higher the molar ratio of metal compound to cyclic ether monomer originally present, the lower the molecular weight of the resulting polyether will be. Similarly, the higher the ratio of accelerator (if present) to monomer originally present, the lower the molecular weight of the polyether will be. It is believed the effects of these two ratios are cumulative. For these effects see Examples 7 and 8.

The polymerization may be run at a temperature of about -80°C to about 130°C. If this temperature is above the boiling point of the cyclic ether monomer, a pressure vessel may be used. A preferred temperature is ambient to the boiling point of the monomer, or 110°C, whichever is lower. An inert solvent such as di-n-butyl ether, diethyl ether or toluene may be used, but it is preferred if solvents are not present. Protic compounds such as water, methanol and ethanol should preferably not be present, and it is convenient to exclude them by drying the starting materials and keeping the process under an inert dry gas such as nitrogen. As in most chemical processes, the ingredients should be mixed at least initially. Continued agitation is preferred to assure that the process materials remain well mixed, and to avoid overheating. The polymerization is mildly exothermic. If the polymerization temperature goes up appreciably, refluxing of the monomer may be used to help cool the process.

The polymers produced often have polydispersities significantly less than 2, which is possibly indicative of a "Living polymerization". Also indicative of this is the fact that as the polymerization proceeds, the molecular weight, particularly the number average molecular weight, increases.

This invention is concerned with the depolymerization of a polymer consisting essentially of the repeat unit -[CHR¹CR²R³CR²R³CHR⁴O]- wherein R¹, R², R³, and R⁴ are defined above, and preferred combinations are as given above for the polymerization process when n=2. A catalyst designated MZₛ·Qₜ is used, wherein M, S, Q, t, and Z, and their preferred embodiments, are as given above.

The depolymerization process is carried out at about 100°C to about 250°C, preferably about 130° to about 200°C. Although air can be used to blanket the process, it is preferred to use an inert atmosphere such as nitrogen to avoid possible side reactions. The polytetrahydrofuran need not be dried before use. A solvent may be used, but it is preferred to carry out the process without solvent.

The amount of catalyst compared to polyether present is not critical, 0.1-15% by weight being useful, preferably about 1 to 3% by weight of catalyst.

The depolymerization process may be carried out by just heating the polyether in the presence of the catalyst. In order to avoid boiling off the often volatile tetrahydrofurans, a pressure vessel may be needed. However, it is preferred to carry out the depolymerization while constantly distilling off the (substituted) tetrahydrofuran as it forms. It is believed that this ensures driving this process to produce the monomeric tetrahydrofuran. The recovered monomeric tetrahydrofuran may be used in the polymerization to form a polytetrahydrofuran.

Both the polymerization and depolymerization processes can be done in a variety of ways known to the artisan. The polymerization can be done by batch, semi-batch and continuous processes. Continuous processes include continuous stirred tank reactor(s) with one or more stages, and/or plug flow reactors (see Example 19). The depolymerization process can also be done by similar methods. In this process, a continuous process could be constant addition of polyether to the reactor, while distilling off a similar amount of a monomeric tetrahydrofuran. Other variations will be evident to one skilled in this art.

In both the polymerization and depolymerization processes disclosed herein the catalyst may be recovered and reused in either process. It may be recovered from the polymerization process by extracting the polymer formed with water, while it can be recovered from the depolymerization process by extracting the distillation residue with water. In both instances, the recovered catalyst may be used again in the polymerization or depolymerization processes. In both instances the aqueous washings may be concentrated by removal of the water (as by evaporation) and the solid catalyst recovered. See Examples 4 and 5 for reuse of catalyst.

In the Examples, the following abbreviations are used:
GPC - gel permeation chromatography
Nation™ - a sulfonated perfluoropolymer produced by E. I. du Pont de Nemours & Co., Wilmington, DE, USA
Mn - number average molecular weight
Mw - weight average molecular weight
RB - round bottom
PD - polydispersity (Mw/Mn)
PS - polystyrene
SS - stainless steel
STD - standard
Tf - triflate

### EXAMPLE 1

### Preparation of Didymium (Mischmetall) Triflate

Didymium (mischmetall) oxide (17 g) and water (50 mL) were added to a 200 mL RB flask equipped with stirring bar and an addition funnel and reflux condenser. Triflic acid (50 g) was slowly added via the addition funnel to the resulting stirred slurry. After the addition was completed a homogeneous solution resulted, thus an additional 2.0 g of the oxide was added and the slurry heated to reflux for 2 hrs. The cooled slurry was filtered, the filtrate concentrated at reduced pressure and then dried under vacuum at 150-210°C affording 58.4 g of a pink solid.

### EXAMPLE 2

### Preparation of Ytterbium Nafion® Salt

In a 300 mL RB flask were added ytterbium oxide (0.75 g) and Nafion® perfluorinated ion exchange resin powder (300 mL, 5 wt. % solution in a mixture of lower aliphatic alcohols and 10% water). The resulting mixture was heated to 100°C and stirred overnight. The resulting solution was filtered and dried under vacuum at 150°C, affording 9.21 g of a light brown solid.

### EXAMPLE 3

### Depolymerization of PolyTHF with Yttrium Triflate

Polytetrahydrofuran 1000 (300 g, Aldrich) and yttrium triflate (9 g) were placed in a 500 mL three neck flask equipped with a stirring bar, Vigreaux column (30.5 cm) and a fractional distillation head. A nitrogen purge was then attached and all other openings glass stoppered. The resulting mixture was then heated by an oil bath and the water clear distillate fractions collected as follows:

| Fraction | Oil Bath Temp (°C) | Head Temp (°C) | Weight |
|---|---|---|---|
| 1 | 171-175 | 64.5 | 67.49 |
| 2 | 176 | 64.5 | 71.84 |
| 3 | 176 | 64.5 | 32.84 |
| 4 | 178 | 64.5 | 58.67 |
| 5 | 178 | 64.5 | 56.71 |
| Total weight of distillate collected: 287.55 ¹H NMR analyses of all five fractions confirmed the product to be THF. Yield (Recovery): 95.85% | | | |

### EXAMPLE 4

### Depolymerization of Poly-THF with Yttrium Triflate: Reuse of Catalyst

To the residue remaining from Example 3 was added polytetrahydrofuran 1000 (300 g, Aldrich). The apparatus was reassembled the resulting mixture heated by an oil bath, and the resulting water clear distillate fractions were collected as follows:

| Fraction | Oil Bath Temp (°C) | Head Temp (°C) | Weight |
|---|---|---|---|
| 1 | 170-174 | 63-64 | 43.39 |
| 2 | 174 | 64 | 62.68 |
| 3 | 175 | 65 | 66.15 |
| 4 | 177 | 65 | 55.15 |
| 5 | 177 | 65 | 32.58 |
| Total weight of distillate collected: 259.95 g Yield (Recovery): 86.65% Total time elapsed from start of collection to termination of Example: 2 hrs. 50 mins. | | | |

### EXAMPLE 5

### Recovery of Catalyst from Depolymerization

Water (100 mL) was added to the residue from Example 4, the resulting mixture was stirred at room temperature for approximately 1 hour, the aqueous phase separated and concentrated at reduced pressure and dried in vacuo at 180°C overnight affording a brown solid. This solid was again dissolved in water, then filtrated, the filtrated concentrated at reduced pressure. The resulting solid was dried under vacuum at 180°C overnight affording a cream solid: 6.48 g (72%) of recovered catalyst.

### EXAMPLE 6

### Depolymerization of Poly-THF/3-Methyl-THF Copolymer with Yttrium Triflate

Poly-tetrahydrofuran/3-methyl-tetrahydrofuran copolymer (308.6 g) containing 3385 ppm water and yttrium triflate (9 g) were placed in a 500 mL three neck flask equipped with a stirring bar, Vigreaux column (30.5 cm), a thermometer and a fractional distillation head. A nitrogen purge was attached and all other opening glass stoppered. The resulting mixture was heated by an oil bath and the water clear distillate fractions collected as follows:

| Fraction | Oil Bath Temp. (°C) | Rxn. Temp. (°C) | Head Temp. (°C) | Weight (g) |
|---|---|---|---|---|
| 1 | 180-182 | 140-145 | 65-70 | 64.35 |
| 2 | 182-184 | 140 | 69-70 | 71.03 |
| 3 | 183-185 | 140-144 | 70 | 69.35 |
| 4 | 185 | 143 | 70 | 70.12 |
| 5 | 185 | - | 70 | 22.35 |
| Total Weight Collected: 297.20 g % Yield (Recovery): 96.47 Total depolymerization time: 2 hrs. 25 mins. | | | | |

### EXAMPLE 7

### Depolymerisation of PolyTHF, Diacetate Capped, with Yttrium Triflate

Polytetrahydrofuran which was diacetate capped (300 g, Mn 1850) and yttrium triflate (9 g) were placed in a 500 mL three flask equipped with a stirring bar, Vigreaux (30.5 cm), a thermometer and a fractional distillation head. A nitrogen purge was attached and all other openings glass stoppered. The resulting mixture was heated by an oil bath and the water clear distillate fractions collected as follows:

| Fraction | Oil Bath Temp. (°C) | Rxn. Temp. (°C) | Head Temp. (°C) | Weight (g) |
|---|---|---|---|---|
| 1 | 158-160 | 105-129 | 64 | 82.78 |
| 2 | 160-161 | 116-129 | 64-66 | 62.91 |
| 3 | 161 | 116 | 64-67 | 77.71 |
| 4 | 161-180 | - | 67-69 | 51.50 |
| Total weight Collected: 274.90 g % Yield (Recovery): 91.63 Total depolymerization time: 1 hr. 25 mins. | | | | |

### EXAMPLE 8

### Preparation of Bis(n-cyclopentadienyl)tetrahydrofuran-bis(trifluoromethanesulfonato)hafnium

In a dry box, hafnocene dichloride (9.93 g) was dissolved in THF (300 mL). To this solution, with stirring, was added a solution of silver triflate (14.12 g) in THF (100 mL). After 10 minutes the precipitated silver chloride was filtered off and the resulting filtrate concentrated to approximately half its volume at reduced pressure. Hexane (250 mL) was added and the resulting mixture placed in the freezer. The resulting precipitate was filtered and then dried under vacuum. Yield: 10.02 g. ¹H NMR (CDCl₃): 6.68 (s, 10 H), 3.76 (m, 4H), 1.84 (m, 4H).

### EXAMPLE 9

### Preparation of Bis(pentamethyl-n-cyclopentadienyl)-bis(trifluoromethanesulfonato)zirconium

In a dry box, bis(pentamethylcyclopentadienyl)-zirconium dichloride (10.00 g) was dissolved in THF (300 mL). To this solution, with stirring, was added a solution of silver triflate (12.46 g) in THF (100 mL). After 15 minutes the precipitated silver chloride was filtered off and the resulting filtrate concentrated to approximately half its volume at reduced pressure. Hexane (250 mL) was added and the resulting mixture placed in the freezer. The resulting yellow precipitate was filtered and then dried under vacuum. Yield: 6.02 g. ¹H NMR (CDCl₃) : 2.12 (s).

### EXAMPLE 10

### Preparation of Bis(n-cyclopentadienyl)-bis (trifluoromethanesulfonato)vanadium

In a dry box, vanadocene dichloride (5.00 g) was dissolved in THF (300 mL). To this solution, with stirring, was added a solution of silver triflate (11.19 g) in THF (100 mL). After 15 minutes the precipitated silver chloride was filtered off and the resulting filtrate concentrated to approximately half its volume at reduced pressure. Hexane (250 mL) was added and the resulting mixture placed in the freezer. The resulting green precipitate was filtered and then dried under vacuum. Yield: 6.99 g.

### EXAMPLE 11

### Depolymerization of Polytetrahydrofuran with Copper Triflate

Polytetrahydrofuran diol, Mn = ∼1,000, and copper triflate (9 g) were placed in a 500 mL three neck flask equipped with a stirring bar, Vigreaux column (12") and a fractional distillation head. A nitrogen purge was attached and all other openings were glass stoppered. The resulting mixture was heated by an oil bath and the resulting water clear distillate fractions collected as follow:

| Fraction | Oil Bath Temp (°C) | Rxn Temp. (°C) | Head Temp. (°C) | Weight (g) |
|---|---|---|---|---|
| 1 | 168 | 135-139 | 64 | 47.85 |
| 2 | 168 | 128-135 | 64 | 57.09 |
| 3 | 168 | 118-128 | 66 | 53.67 |
| 4 | 168 | 106-128 | 66 | 57.77 |
| 5 | 168 | 106 | 66 | 76.30 |
| Total weight of distillate collected: 292.68 g % Yield (Recovery): 97.56% Total depolymerization time from start of collection to termination of experiment: 1 hr. 45 mins. | | | | |

### EXAMPLE 12

### Preparation of Bismuth Triflate

BiCl₃ (630 mg, 2 mmol) was slurried in CH₂Cl₂ (20 mL). Triflic acid (900 mg, 6 mmol) was added dropwise, and the mixture was stirred overnight at room temperature. The solvent was removed to give 0.9 g of an off white solid. ¹⁹F NMR (DMSO-d₆): δ -77.3.

### EXAMPLE 13

### Preparation of Zr(OSO₂CF₃)_{4.}Zr(OCOCH₃)₄

Solid Zr(OTf)₄ (0.5 g) and Zr(CF₃CO₂)₄ (0.5 g) were mixed and THF (25 mL) was added. The mixture was stirred for 15 minutes at room temperature. The solvent was removed and 0.9 g of off white solid was collected. ¹⁹F NMR (CDCl₃): δ -78.3, -76.2 (Zr(CF₃CO₂)₄ comes at δ -75.8).

### EXAMPLE 14

### Preparation of Gold Triflate

AuBr₃ (0.90 g, 2.1 mmol) was slurried in CH₂Cl₂ (20 mL) and triflic acid (0.90 g, 6.3 mmol) was added dropwise. The mixture was stirred overnight at room temperature. The solvent was removed and 0.77 g of black solid was collected. ¹⁹F NMR (DMSO-d₆): δ -76.9.

### EXAMPLE 15

### Preparation of Y(OSO₂CF₃)₂Cl

Solid Y(OTf)₃ (540 mg, 1 mmol) and YCl₃ (98 mg, 0.5 mmol) were mixed, and this mixture was poured into stirred THF (30 mL). The mixture became warm as the solid dissolved. The solution was stirred for 15 min, and the THF was removed. ¹⁹F NMR (DMSO-d₆): δ -77.3.

### EXAMPLE 16

### Preparation of Y(OSO₂CF₃)Cl₂

Solid Y(OTf)₃ (540 mg, 1 mmol) and YCl₃ (390 mg, 2 mmol) were mixed, and this mixture was poured into stirred THF (30 mL). The mixture became warm as the solid dissolved. The solution was stirred for 15 min, and the THF was removed. ¹⁹F NMR (DMSO-d₆): δ -77.2

### EXAMPLE 17

### Preparation of Ta(OSO₂CF₃)₄OCH₂CH₃

Ta(OEt)₅ (813 mg, 2 mmol) was dissolved in CH₂Cl₂ (20 mL). Triflic acid (1.5 g, 10 mol) was added dropwise and the solution stirred overnight at room temperature. The solvent was removed to produce a colorless oil. ¹H and ¹⁹F NMR show a mixture of compounds. ¹H NMR (CDCl₃): δ 1.85 (t), 1.9 (t), 4.1, (q), 4.15 (broad, q).

### EXAMPLE 18

### Preparation of Iron(III) Bis-triflate-acetylacetonate

Fe(acac)₃ (1.0 g, 2.8 mmol) was dissolved in CH₂Cl₂ (15 mL), and triflic acid (850 mg, 5.7 mmol) was added dropwise. The purple solution was stirred overnight at room temperature. The solvent was removed to give a dark oil.

### EXAMPLE 19

### Preparation of Ruthenium(TIT) Triflate

RuCl₃ (1.0 g, 4.6 mmol) was slurried in CH₂Cl₂ (20 mL) and triflic acid (2.0 g, 13.6 mmol) was added dropwise. The mixture was stirred at room temperature overnight. The solvent was removed and 1.15 g of black solid was collected. ¹⁹F NMR (CDCl₃): δ -76.7.

### EXAMPLE 20

### Preparation of Palladium(II) Triflate

PdCl₂ (1.0 g, 5.6 mmol) was slurried in CH₂Cl₂ (20 mL) and triflic acid (1.7 g, 11.3 mmol) was added dropwise. The mixture was stirred at room temperature overnight. The solvent was removed and 0.9 g of rust color solid was collected. ¹⁹F NMR (CDCl₃): δ -78.5.

### EXAMPLE 21

### Preparation of Rhenium(V) Triflate

ReCl₅ (1.0 g, 2.75 mmol) was slurried in CH₂Cl₂ (25 mL) and triflic acid (2.1 g, 13.7 mmol) was added dropwise. The mixture was stirred overnight at room temperature. The solvent was removed and 0.9 g of black solid was collected. ¹⁹F NMR (CDCl₃): δ -74.4, -76.3 (small peak).

### EXAMPLE 22

### Preparation of Chromium(II) Triflate

CrCl₂ (0.62 g, 5 mmol) was slurried in CH₂Cl₂ (20 mL) and triflic acid (2.3 g, 15 mmol) was added dropwise. The mixture was stirred overnight at room temperature. The solvent was removed and 1.25 g of gray solid was collected. ¹⁹F NMR (DMSO-d₆): **δ** -76.65, -76.72.

### EXAMPLE 23

### Preparation of n-Cyclopentadienyl-tris(trifluoromethanesulfonato)zirconium

Cp*ZrCl₃ (1.0 g, 3 mmol) was slurried in CH₂Cl₂ (40 mL). THF (10.00 mL) was added to dissolve the yellow solid. Solid AgOTf (2.3 g, 9 mmol) was added; a white solid formed immediately. The mixture was stirred 15 min, and the orangish solution was filtered. The solvent was removed to produce an orangish oil. The material was crystallized from ether and 1.1 g of yellow solid was collected. ¹H NMR showed several peaks around 2 ppm. Coordinated THF is observed by ¹H NMR (near 8 3.5 and 1.2).

### EXAMPLE 24

### Preparation of Strontium Triflate

SrCl₂ (790 mg, 5 mmol) was slurried in CH₂Cl₂ (20 mL) and triflic acid (1.5 g, 10 mmol) was added dropwise. The mixture was stirred overnight at room temperature. The solvent was removed and 1.7 g of white solid was collected. ¹⁹F NMR (DMSO-d₆): δ -77.4.

### EXAMPLE 25

### Preparation of Cp₂(OTf)Zr-O-Zr(OTf)Cp₂

Cp₂Zr(Cl)-O-(Cl)ZrCp₂ (1.3 g, 2.5 mmol) was dissolved in CH₂Cl₂ (40 mL), and AgOTf (1.3 g, 5 mmol) was added. The mixture was stirred over a weekend. The mixture was filtered and the solvent was removed. A white solid formed as the solvent evaporated. The mixture was filtered and the solid was washed with Et₂O. 1.4 g of white solid was collected. ¹H NMR (toluene-d₈) : δ 6.0 (s).

### EXAMPLE 26

### Preparation of Bis-(n-Cyclopentadienyl)-bis(trifluoromethanesulfonato)molybdenum

Solid Cp₂MoCl₂ (500 mg, 1.7 mmol) and AgOTf (0.91 g, 3.5 mmol) were mixed and CH₂Cl₂ (30 mL). The mixture stirred overnight at room temperature. The white solid was filtered off and the solvent was evaporated to give 300 mg of a green solid. ¹H NMR (CDCl₃) : δ 6.4 (s).

### EXAMPLE 27

### Preparation of Bis(trifluoromethanesulfonato)-bis(acetylacetonate)zirconium

Zr(acac)₄ (1.46 g, 3 mmol) was dissolved in CH₂Cl₂ (5 mL). A solution of triflic acid (0.9 g, 6 mmol) in CH2Cl₂ (1 mL) was added to the Zr(acac)₄ solution. The solution was stirred 2 hours at room temperature. The solvent was removed and 1.79 g of yellow solid was collected. ¹⁹F NMR (CDCl₃) : δ -78.4; ¹H NMR (CDCl₃) : δ 2.15 (s), 5.82 (broad).

### EXAMPLE 28

### Preparation of Yttrium Bis(trifluoromethanesulfonato)-2,2,6,6-tetramethyl-3,5-heptanedionate

(t-Buacac)₃Y (0.64 g, 1 mmol) was dissolved in CH₂Cl₂ (5 mL). A solution of triflic acid (0.3 g, 2 mmol) in CH₂Cl₂ (1 mL) was added and the solution was stirred 2 hours at room temperature. The solvent was removed and a white solid was collected. ¹H NMR (CDCl₃) : δ 1.2, 1.1 (broad s); ¹⁹F NMR (CDCl₃) : δ -78.4.

### EXAMPLE 29

### Preparation of Yttrium Trifluoromethanesulfonato-bis(2,2,6,6-tetramethyl-3,5-heptanedionate)

(t-Buacac)₃Y (0.64 g, 1 mmol) was dissolved in CH₂Cl₂ (5 mL). A solution of triflic acid (0.15 g, 1 mmol) in CH₂Cl₂ (1 mL) was added dropwise and the solution was stirred 2 h at room temperature. The solvent was removed and a white solid was collected. ¹H NMR (CDCl₃): δ 1.15, 1.02; ¹⁹F NMR (CDCl₃) : δ -78.6 (small, broad), -76.7.

### EXAMPLE 30

### Preparation of VO(OTf)ₙ(OCHMe₂)₃₋ₙ

V(O)(OnPr)₃ (1.2 g, 5 mmol) was dissolved in CH₂Cl₂ (30 mL). Triflic acid (2.2 g, 15 mmol) was added dropwise to produce a dark red solution. The solvent was removed and a dark oil was produced.

### EXAMPLE 31

### Preparation of Silicon Triflate

SiCl₄ (3 g, 17.6 mmol) was dissolved in CH₂Cl₂ (75 mL) and triflic acid (10.6 g, 70.7 mmol) was added dropwise. The mixture was stirred at room temperature over the weekend. The solvent was removed and 4.8 g of brown liquid was collected. ¹⁹F NMR (DMSO-d₆): δ -76.4 (intense), small broad peaks at -77.8 and -77.95.

## Claims

1. A process for the depolymerization of a polyether to a tetrahydrofuran, comprising, contacting at a temperature of about 100°C to about 250°C, a polymer consisting essentially of one or more repeat units of the formula
-[CHR¹CR²R³CR²R³CHR⁴O]-
with a compound of the formula MZₛQₜ, wherein:
each R¹, R², R³ and R⁴ is independently hydrogen or hydrocarbyl containing 1 to 20 carbon atoms;
M is a metal selected from the group consisting of cobalt, vanadium, niobium, tungsten, strontium, barium, scandium, yttrium, thulium and the other rare earth metals, titanium, zirconium, hafnium, chromium, molybdenum, tantalum, rhenium, iron, ruthenium, osmium, rhodium, iridium, palladium, platinum, gold, zinc, cadmium, mercury, copper, mischmetall, indium, silicon, germanium, tin, lead, arsenic, antimony and bismuth;
at least one of Z is an anion of the formula -OSO₂R⁵, wherein R⁵ is perfluoroalkyl containing 1 to 12 carbon atoms or part of a fluorinated polymer wherein the carbon atoms alpha beta to the sulfonate group are together bonded to at least four fluorine atoms, or tetraphenylborate, and the remainder of Z is oxo or one or more monovalent anions;
s is 2 when M is copper, strontium, barium, cobalt, rhodium, iridium, palladium, platinum, chromium, zinc, cadmium or mercury;
s is 3 when M is scandium, yttrium, thulium or other rare earth metal, arsenic, antimony, bismuth, gold, iron, ruthenium, osmium, indium or mischmetall;
s is 4 when M is titanium, zirconium, hafnium, molybdenum, silicon, germanium, tin, or lead;
s is 5 when M is rhenium, vanadium, niobium or tantalum;
s is 6 when M is tungsten;
Q is a neutral ligand;
t is 0 or an integer of 1 to 6;
and provided that each oxo group present as part of Z is considered to account for two of s.

2. The process as recited in claim 1 wherein R¹ and R⁴ are each hydrogen, and all of R² and R³ are hydrogen.

3. The process as recited in claim 1 wherein R¹ and R⁴ are each hydrogen, one of R² is hydrogen, the other R² is methyl, and both R³ are hydrogen.

4. The process as recited in claim 1, 2 or 3 wherein R⁵ is trifluoromethyl.

5. The process as recited in any of claims 1 to 4 wherein said temperature is about 130°C to about 200°C.

6. The process as recited in any of claims 1 to 5 wherein said compound of formula MZₛQT is about 1 to about 3% by weight of said polyether.

7. The process as recited in any of claims 1 to 6 wherein M is strontium, scandium, yttrium, thulium or one of the other rare earth metals, titanium, zirconium, hafnium, vanadium, niobium, tantalum, chromium, molybdenum, tungsten, rhenium, iron, ruthenium, palladium, copper, gold, zinc, tin, bismuth or mischmetall.

8. The process as recited in any of claims 1 to 7 wherein all of Z is an anion of the formula ⁻OSO₂R⁵, and wherein R⁵ is trifluoromethyl.

## Patentansprüche

1. Verfahren für die Depolymerisation eines Polyethers zu einem Tetrahydrofuran, umfassend das Kontaktieren bei einer Temperatur von etwa 100° bis etwa 250°C eines Polymers, im Wesentlichen bestehend aus einer oder mehreren repetierenden Einheiten der Formel
-[CHR¹CR²R³CR²R³CHR⁴O]-
mit einer Verbindung der Formel MZₛ·Qₜ, worin sind:
jedes R¹, R², R³ und R⁴ unabhängig Wasserstoff oder Hydrocarbyl mit 1 bis 20 Kohlenstoffatomen;
M ist ein Metall, ausgewählt aus der Gruppe, bestehend aus Cobalt, Vanadium, Niob, Wolfram, Strontium, Barium, Scandium, Yttrium, Thulium und den anderen Seltenerdmetallen, Titan, Zirconium, Hafnium, Chrom, Molybdän, Tantal, Rhenium, Eisen, Ruthenium, Osmium, Rhodium, Iridium, Palladium, Platin, Gold, Zink, Cadmium, Quecksilber, Kupfer, Mischmetalle, Indium, Silicium, Germanium, Zinn, Blei, Arsen, Antimon und Bismut;
mindestens eines von Z ist ein Anion der Formel -OSO₂R⁵, worin R⁵ Perfluoralkyl ist, das 1 bis 12 Kohlenstoffatome enthält, oder ein Teil eines fluorierten Polymers, worin die Kohlenstoffatome, alpha, beta zu der Sulfonat-Gruppe zusammen mindestens an 4 Fluoratomen gebunden sind, oder Tetraphenylborat und der Rest von Z Oxo ist oder eines oder mehrere einwertige Anionen;
s ist 2, wenn M Kupfer ist, Strontium, Barium, Cobalt, Rhodium, Iridium, Palladium, Platin, Chrom, Zink, Cadmium oder Quecksilber;
s ist 3, wenn M Scandium ist, Yttrium, Thulium oder ein Seltenerdmetall, Arsen, Antimon, Bismut, Gold, Eisen, Ruthenium, Osmium, Indium oder Mischmetall;
s ist 4, wenn M Titan ist, Zirconium, Hafnium, Molybdän, Silicium, Germanium, Zinn oder Blei;
s ist 5, wenn M Rhenium ist, Vanadium, Niob oder Tantal;
s ist 6, wenn M Wolfram ist;
Q ist ein neutraler Ligand;
t ist Null oder eine ganze Zahl von 1 bis 6;
und unter der Voraussetzung, dass jede Oxo-Gruppe, die als Teil von Z vorhanden ist, so genommen wird, dass sie für 2 s zählt.

2. Verfahren nach Anspruch 1, bei welchem R¹ und R⁴ Wasserstoff sind und alle R² und R³ Wasserstoff sind.

3. Verfahren nach Anspruch 1, worin R¹ und R⁴ jedes Wasserstoff sind, eines von R² Wasserstoff ist, das andere R² Methyl ist und beide R³ Wasserstoff sind.

4. Verfahren nach Anspruch 1, 2 oder 3, worin R⁵ Trifluormethyl ist.

5. Verfahren nach einem der Ansprüche 1 bis 4, worin die Temperatur etwa 130° bis etwa 200°C beträgt.

6. Verfahren nach einem der Ansprüche 1 bis 5, worin die Verbindung der Formel MZₛ·Qₜ etwa 1% bis etwa 3 Gew.% des Polyethers ausmacht.

7. Verfahren nach einem der Ansprüche 1 bis 6, worin M Strontium ist, Scandium, Yttrium, Thulium oder eines der anderen Seltenerdmetalle, Titan, Zirconium, Hafnium, Vanadium, Niob, Tantal, Chrom, Molybdän, Wolfram, Rhenium, Eisen, Ruthenium, Palladium, Kupfer, Gold, Zink, Zinn, Bismut oder Mischmetall.

8. Verfahren nach einem der Ansprüche 1 bis 7, worin alle Z ein Anion der Formel -OSO₂R⁵ sind und worin R⁵ Trifluormethyl ist.

## Revendications

1. Procédé pour la dépolymérisation d'un polyéther en un tétrahydrofuranne, comprenant la mise en contact à une température d'environ 100°C à environ 250°C d'un polymère constitué essentiellement d'une ou de plusieurs unités de répétition de la formule:
-[CHR¹CR²R³CR²R³CHR⁴O]-
avec un composé de la formule MZₛ·Qₜ, dans lesquelles formules:
chaque R¹, R², R³ et R⁴ est indépendamment un hydrogène ou un groupe hydrocarbyle contenant de 1 à 20 atomes de carbone;
M est un métal choisi dans le groupe constitué de cobalt, de vanadium, de niobium, de tungstène, de strontium, de baryum, de scandium, d'yttrium, de thulium et d'autres métaux des terres rares, de titane, de zirconium, d'hafnium, de chrome, de molybdène, de tantale, de rhénium, de fer, de ruthénium, d'osmium, de rhodium, d'iridium, de palladium, de platine, d'or, de zinc, de cadmium, de mercure, de cuivre, de mischmétal, d'indium, de silicium, de germanium, d'étain, de plomb, d'arsenic, d'antimoine et de bismuth;
au moins un des Z est un anion de la formule -OSO₂R⁵, dans laquelle R⁵ est un groupe perfluoroalkyle contenant de 1 à 12 atomes de carbone ou une partie d'un polymère fluoré dans lequel les atomes de carbone alpha, bêta par rapport au groupe sulfonate sont liés ensemble à au moins quatre atomes de fluor, ou tétraphénylborate, et le reste des Z est un groupe oxo ou un ou plusieurs anions monovalents;
s est 2 lorsque M est le cuivre, le strontium, le baryum, le cobalt, le rhodium, l'iridium, le palladium, le platine, le chrome, le zinc, le cadmium ou le mercure;
s est 3 lorsque M est le scandium, l'yttrium, le thulium ou un autre métal des terres rares, l'arsenic, l'antimoine, le bismuth, l'or, le fer, le ruthénium, l'osmium, l'indium ou un mischmétal;
s est 4 lorsque M est le titane, le zirconium, l'hafnium, le molybdène, le silicium, le germanium, l'étain ou le plomb;
s est 5 lorsque M est le rhénium, le vanadium, le niobium ou le tantale;
s est 6 lorsque M est le tungstène;
Q est un ligand neutre;
t est 0 ou un nombre entier de 1 à 6;
et à condition qu'il soit considéré que chaque groupe oxo présent comme partie de Z constitue deux de s.

2. Procédé suivant la revendication 1, dans lequel R¹ et R⁴ sont chacun un hydrogène et tous les R² et R³ sont des hydrogènes.

3. Procédé suivant la revendication 1, dans lequel R¹ et R⁴ sont chacun un hydrogène et l'un des R² est un hydrogène, l'autre R² est un groupe méthyle et les deux R³ sont des hydrogènes.

4. Procédé suivant la revendication 1, 2 ou 3, dans lequel R⁵ est un groupe trifluorométhyle.

5. Procédé suivant l'une quelconque des revendications 1 à 4, dans lequel ladite température est d'environ 130°C à environ 200°C.

6. Procédé suivant l'une quelconque des revendications 1 à 5, dans lequel ledit composé de la formule MZₛ·Qₜ est d'environ 1 à environ 3% en poids dudit polyéther.

7. Procédé suivant l'une quelconque des revendications 1 à 6, dans lequel M est le strontium, le scandium, l'yttrium, le thulium ou un des autres métaux des terres rares, le titane, le zirconium, l'hafnium, le vanadium, le niobium, le tantale, le chrome, le molybdène, le tungstène, le rhénium, le fer, le ruthénium, le palladium, le cuivre, l'or, le zinc, l'étain, le bismuth ou un mischmétal.

8. Procédé suivant l'une quelconque des revendications 1 à 7, dans lequel la totalité des Z est un anion de la formule -OSO₂R⁵ et dans lequel R⁵ est un groupe trifluorométhyle.
